# EUROPEAN PATENT SPECIFICATION

(11) **EP 3 970 722 B1**
(45) Date of publication and mention of the grant of the patent: **02.12.2026**
(21) Application number: 21197857.2
(22) Date of filing: 20.09.2021
(51) Int. Cl.: A61K 31/4415, A61K 31/51, A61K 31/714, A61K 9/20, A61K 9/24, A61K 9/28, A61K 31/385

(54) **A PHARMACEUTICAL BILAYER TABLET COMPRISING ALPHA LIPOIC ACID AND AT LEAST ONE B VITAMIN**
PHARMAZEUTISCHE ZWEISCHICHTIGE TABLETTE MIT ALPHA-LIPONSÄURE UND MINDESTENS EINEM B-VITAMIN
COMPRIMÉ PHARMACEUTIQUE BICOUCHE COMPRENANT DE L'ACIDE ALPHA LIPOÏQUE ET AU MOINS UNE VITAMINE B

(30) Priority: 21.09.2020 TR 202014936
(43) Date of publication of application: 23.03.2022
(73) Proprietor: Sanovel Ilac Sanayi ve Ticaret A.S., Sisli - Istanbul (TR)
(72) Inventor: SUNEL, Fatih, Istanbul (TR); PEHLIVAN AKALIN, Nur, Istanbul (TR); KARACA AYVAZ, Gizem, Istanbul (TR); SARI, Selda, Istanbul (TR)
(74) Representative: Genç Ilhan, Oznur

(56) References cited:
- EP-A1- 3 366 291
- WO-A2-2010/115947
- IT-A1- MI20 102 397

## Description

### Field of the Invention

The present invention relates to a pharmaceutical bilayer tablet comprising alpha lipoic acid in combination with thiamine hydrochloride, pyridoxine hydrochloride and cyanocobalamin. Further, the present invention also relates to a simple, rapid, cost effective, time-saving and industrially convenient process. The bilayer tablet of the present invention is manufactured as an extended release tableted dietary supplement and are intended as daily supplements.

### Background of the Invention

Vitamins and minerals may be broadly defined as substances that are essential for the maintenance of normal metabolic function but are not synthesized in the body. These substances must be furnished from an exogenous source. A healthy individual ingesting a well-balanced diet receives adequate amounts of vitamins and minerals from food. Vitamins obtained in this manner are not generally regarded as drugs.

Recent studies have illustrated the important physiological roles played by vitamins and established a correlation between deficiencies or excesses of these nutrients and the etiologies of certain disease states in humans.

Multiple-vitamin therapy, using supplemental dietary multi-vitamins, is desirable in a variety of situations. Since deficiency of a single vitamin is rarely encountered clinically, it is customary practice in cases of suspected vitamin deficiency, as well as in prophylactic treatment, to recommend that multiple-vitamin therapy be administered.

Alpha lipoic acid (ALA) is an organosulfur compound derived from octanoic acid. ALA is made in animals normally, and is essential for aerobic metabolism. It is also manufactured and is available as a dietary supplement in some countries where it is marketed as an antioxidant, and is available as a pharmaceutical drug in other countries.

It is able to pass the blood-brain barrier and is putatively used for detoxification of mercury attached to the brain cells. It can mobilize bound mercury into the blood stream as it is a mercaptan (sulfur compound which readily binds to the mercury). ALA and perhaps vitamin B12 could make it possible for other chelators to remove mercury safely out of the body and could perhaps one day be used as a treatment for autism. Because ALA is related to cellular uptake of glucose and it is both soluble in water and fat, it is being used for treatment in diabetes. It may be helpful for people with Alzheimer's disease or Parkinson's disease.

Orally-administered tablets of alpha lipoic acid (thioctic acid) are commercially available under the name Thioctacid^{®}. Its chemical name is (R)-5-(1,2-Dithiolan-3-yl)pentanoic acid and it has the chemical structure shown in Formula I.

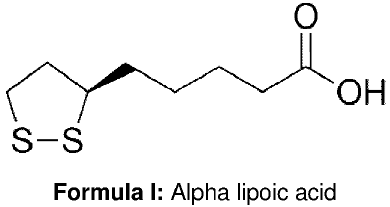

On the other hand, vitamins are classified as either fat soluble (vitamins A, D, E and K) or water soluble (vitamins B and C). This difference between the two groups is very important since it determines how each vitamin acts within the body.

B vitamins are a class of water-soluble vitamins that play important roles in cell metabolism. Though these vitamins share similar names, research shows that they are chemically distinct vitamins that often coexist in the same foods.

Thiamine, also known as vitamin B1, is a vitamin found in food and used as a dietary supplement. As a supplement it is used to treat and prevent thiamine deficiency and disorders that result from it including beriberi and Korsakoff's syndrome. Thiamine plays a key role in intracellular glucose metabolism and it is thought that thiamine inhibits the effect of glucose and insulin on arterial smooth muscle cell proliferation. Thiamine plays an important role in helping the body convert carbohydrates and fat into energy. It is essential for normal growth and development and helps to maintain proper functioning of the heart and the nervous and digestive systems. Thiamine cannot be stored in the body; however, once absorbed, the vitamin is concentrated in muscle tissue.

Its chemical name is 3-[(4-amino-2-methylpyrimidin-5-yl)methyl]-5-(2-hydroxyethyl)-4-methyl-1,3-thiazol-3-ium and it has the chemical structure shown in Formula II.

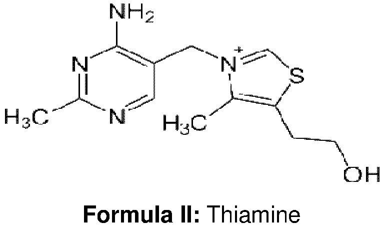

Pyridoxine is the 4-methanol form of vitamin B6 found commonly in food and used as dietary supplement. As a supplement it is used to treat and prevent pyridoxine deficiency, sideroblastic anaemia, pyridoxine-dependent epilepsy, certain metabolic disorders, problems from isoniazid, and mushroom poisoning. It is converted to pyridoxal 5-phosphate in the body. Pyridoxal 5-phosphate is a coenzyme for synthesis of amino acids, neurotransmitters (serotonin, norepinephrine), sphingolipids, aminolevulinic acid.

Its chemical name is 4,5-bis(hydroxymethyl)-2-methylpyridin-3-ol and it has the chemical structure shown in Formula III.

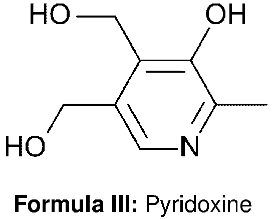

Cyanocobalamin, commonly known as Vitamin B12, is the most chemically complex of all the vitamins. Cyanocobalamin cannot be made by plants or by animals, as the only type of organisms that have the enzymes required for the synthesis of cyanocobalamin are bacteria and archaea. Higher plants do not concentrate cyanocobalamin from the soil and so are a poor source of the substance as compared with animal tissues. Cyanocobalamin is naturally found in foods including meat (especially liver and shellfish), eggs, and milk products. It is usually prescribed after surgical removal of part or all of the stomach or intestine to ensure adequate serum levels of vitamin B12. It is also used to treat pernicious anemia, vitamin B12 deficiency (due to low intake from food), thyrotoxicosis, hemorrhage, malignancy, liver disease and kidney disease.

Its chemical name is [(1R,2R,3S,4S,8S,9S,14S,18R,19R)-4,9,14-tris(2-carbamoylethyl)-3,8,19-tris(carbamoylmethyl)-18-(2-{[(2R)-2-[({[(2R,3S,4R,5S)-5-(5,6-dimethyl-1H-1,3-benzodiazol-1-yl)-4-hydroxy-2-(hydroxymethyl)oxolan-3-yl]oxy}(hydroxy)phosphoryl)oxy] propyl]carbamoyl}ethyl)-2,3,6,8,13,13,16,18-octamethyl-20,21,22,23-tetraazapentacyclo [15.2.1.1²,⁵.1⁷,¹⁰.1^{12,15}]tricosa-5(23),6,10(22),11,15(21),16-hexaen-20-yl]cobaltcarbonitrile and it has the chemical structure shown in Formula IV.

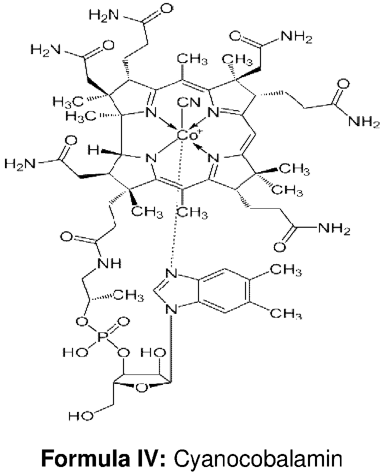

It is a well-known technic to combine vitamins, vitamin-like compounds and minerals in the same dosage form in order to enhance the bioavailability for the same therapeutic effect or in order to come up with a multi-indication dosage form. Combination therapy also reduces the daily dosages to be taken by patients and simplifies dosing schedule thereby increases patient compliance.

Even if it seems easy to combine vitamins with similar water solubilities in one dosage form, it is getting much more complex when it comes to incompatible vitamins and/or vitamin-like compounds and minerals which have highly contradictory water solubilities.

In the state of art, there are various examples presenting pharmaceutical formulations or treatment methods in which vitamins are combined. The patent document numbered EP1267844B2, which is one of these examples, mentions a therapeutic formulation in the form of a beadlet combining vitamin C with Vitamin B1, Vitamin B2, Vitamin B6, Vitamin H, Vitamin PP or pro-Vitamin B5 or mixtures thereof.

In the application numbered CN104546782A, enteric-coated pellets of a lipoic acid formulation are subjected to the invention. The formulation comprises microcrystalline cellulose as disintegrant to provide the required dissolution profile. Besides, it is stated that the amount of the enteric coating is between 20-40% in the composition.

The patent document numbered EP1855653B1 asserts an oral composition comprising various vitamins, minerals and trace elements in three separate parts in a single dosage form. In the document, a controlled release dosage form, in which these separate parts have different release systems, is explicated. Besides, it is claimed that B vitamins are not situated in the same part together.

EP 3 366 291 A1 discloses a multilayer dosage form which comprises alpha lipoic acid and water-soluble vitamins in separate layers in which alpha lipoic acid layer is free of talc and disintegrant.

WO 2010/115947 A2 discloses bilayer modified-release formulations containing alpha-lipoic acid where layers do not comprise disintegrant.

IT MI20 102 397 A1 discloses bilayer tablets where one layer is immediate release and other one is sustained release and only one layer comprises disintegrant.

In this sense, there is still a need for a combination which eliminates especially the water solubility difference problem of B vitamins and which enhances the dissolution profile and bioavailability of the final pharmaceutical product.

### Detailed Description of the Invention

The main object of the present invention is to obtain combination formulations, comprising alpha lipoic acid combination with thiamine hydrochloride (B1), pyridoxine hydrochloride (B6) and cyanocobalamin (B12), according to claim 1, eliminating all aforesaid problems and bringing additional advantages to the relevant prior art.

Another object of the present invention is to provide a bilayer tablet of alpha lipoic acid and thiamine hydrochloride (B1), pyridoxine hydrochloride (B6) and cyanocobalamin (B12). The bilayer tablet has two coating layers which are a film coating layer and an enteric coating.

Another object of the present invention is to obtain a bilayer tablet of alpha lipoic acid and thiamine hydrochloride (B1), pyridoxine hydrochloride (B6) and cyanocobalamin (B12) with high stability and bioavailability.

A further object of the present invention is to develop a bilayer tablet of alpha lipoic acid and thiamine hydrochloride (B1), pyridoxine hydrochloride (B6) and cyanocobalamin (B12) having an improved level of dissolution rate and solubility.

A core tablet as used herein relates to an uncoated tablet, comprising at least one pharmaceutically active compound. A tablet core useful according to the present invention may be in any geometrical form and shape and may be obtained according, e.g. analogously, to a method as conventional.

According to the invention, a pharmaceutical bilayer tablet comprises;
a. first layer comprising thiamine hydrochloride, pyridoxine hydrochloride and cyanocobalamin,
b. second layer comprising alpha lipoic acid

wherein each layer comprising at least one disintegrant and wherein disintegrants are selected from the group of consisting of sodium starch glycolate, low-substitute poloxamer, magnesium aluminum silica, sodium carboxy methyl cellulose, carboxy methyl cellulose calcium, docusate sodium, polyacrylin potassium, sodium glycine carbonate or mixtures thereof,
wherein the amount of the disintegrants is between 1.0% and 15.0% by weight of the total core tablet, wherein the amount of the disintegrants in the first layer is between 1.0% and 10.0% by weight of the total core tablet and the amount of the disintegrants in the second layer is between 1.0% and 5.0% by weight of the total core tablet and,
wherein the disintegrant is sodium starch glycolate.

Having two separate layers in the tablet prevents the incompatibility between alpha lipoic acid and vitamins.

The tablet of the invention comprises sodium starch glycolate as the disintegrant. Further suitable disintegrants may be selected from the group comprising croscarmellose sodium, sodium carbonate, cross-linked polyvinylpyrrolidone (crospovidone), polycarbophil, low-substitue poloxamer, alginic acid and alginates, ion-exchange resins, magnesium aluminum silica, sodium carboxy methyl cellulose, carboxy methyl cellulose calcium, docusate sodium, guar gum, polyacrylin potassium, sodium alginate, sodium glycine carbonate or mixtures thereof.

The dissolution and disintegration of alpha lipoic acid has low solubility and a high amount of alpha lipoic acid is used in the second layer. Using a disintegrant especially on the second layer, it provides the desired dissolution profile throughout the shelf life

According to the invention, the amount of the disintegrants is betweer 1.0% and 15.0% by weight of the total core tablet.

According to the invention, the amount of the disintegrants in the first layer is between 1.0% and 10.0% by weight of the total core tablet and the amount of the disintegrants in the second layer is between 1.0% and 5.0% by weight of the total core tablet.

According to the invention, the disintegrant is sodium starch glycolate. In one embodiment of the invention, the weight ratio of first layer to second layer is between 1.0 and 3.0 or between 1.5 and 2.1.

According to one embodiment of the invention, the amount of thiamine hydrochloride is between 15.0% and 30.0% by weight of the total core tablet. Preferably, the amount of thiamine hydrochloride is between 20.0% and 25.0% by weight of the total core tablet.

According to one embodiment of the invention, the amount of pyridoxine hydrochloride is between 15.0% and 30.0% by weight of the total core tablet. Preferably, the amount of pyridoxine hydrochloride is between 20.0% and 25.0% by weight of the total core tablet.

According to one embodiment of the invention, the amount of cyanocobalamin is between 0.01% and 2.0% by weight of the total core tablet. Preferably, the amount of cyanocobalamin is between 0.01% and 1.0% or between 0.01% and 0.5% by weight of the total core tablet.

According to one embodiment of the invention, the amount of alpha lipoic acid is between 20.0% and 40.0% by weight of the total core tablet. Preferably, the amount of alpha lipoic acid is between 22.0% and 35.0% or between 25.0% and 30.0% by weight of the total core tablet.

According to one embodiment of the invention, a pharmaceutical bilayer tablet comprises;
- 15.0% - 30.0% by weight of thiamine hydrochloride,
- 15.0% - 30.0% by weight of pyridoxine hydrochloride,
- 0.01% - 2.0% by weight of cyanocobalamin,
- 20.0% - 40.0% by weight of alpha lipoic acid in the total core tablet.

According to one embodiment of the invention, the bilayer tablet further comprises at least one pharmaceutically acceptable excipient selected from fillers, binders, lubricants/glidants or mixtures thereof.

Suitable fillers are selected from the group comprising microcrystalline cellulose, lactose monohydrate, lactose, mannitol, spray-dried mannitol, starch, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate, calcium carbonate or mixtures thereof.

According to one embodiment of the invention, the amount of the fillers is between 1.0% and 25.0% by weight of the total core tablet.

According to one embodiment of the invention, the filler is microcrystalline cellulose (PH 101 or 102).

According to one embodiment of the invention, the filler is used in both the first layer and second layer.

Suitable binder are selected from the group comprising hydroxypropyl cellulose (HPC), polyvinylpyrrolidone, copovidone, polyvinylpyrrolidone K30 (povidone K30), carnauba wax, polymethacrylate, glyceryl behenate, carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, xanthan gum, guar gum, alginate, hyaluronic acid, carbomer, cellulose acetate phthalate, hydroxyethyl methyl cellulose, polyacrylamide, aluminum hydroxide, acacia mucilage, polydextrose or mixtures thereof.

According to one embodiment of the invention, the amount of the binders is between 1.0% and 10.0% by weight of the total core tablet.

According to one embodiment of the invention, the binder is hydroxypropyl cellulose.

According to one embodiment of the invention, the binder is used in both the first layer and second layer.

Suitable lubricant/glidants are selected from the group comprising magnesium stearate, colloidal silicon dioxide, sodium lauryl sulfate, sodium stearyl fumarate, zinc stearate, calcium stearate, mineral oil, talc, polyethylene glycol, glyceryl monostearate, glyceryl palmitostearate, magnesium lauryl sulfate, fumaric acid, zinc stearate, stearic acid or mixtures thereof.

According to one embodiment of the invention, the lubricant/glidants is magnesium stearate or colloidal silicon dioxide or mixtures thereof.

According to one embodiment of the invention, the lubricant/glidant is used in both the first layer and second layer.

At this invention, each layer comprises at least two same excipients.

A further advantage of the present invention is to provide less side effects. It has been found that during the process of a bilayer tablet, at least two same excipients has been used for each layer. Also, this helps effective and easy to prepare.

According this preferred embodiment, a pharmaceutical bilayer tablet comprises a core and two coating layers. Preferably, the coating layers are a film coating layer and an enteric coating.

According to the preferred embodiment of the invention, the composition comprises at least one enteric coating to prevent the tablet dissolution or disintegration in the gastric environment.

Suitable coating ingredients are selected from the group comprising hydroxypropyl methylcellulose, triacetin, glycerol triacetate, methacrylic acid / ethyl acrylate copolymer (1:1), lactose monohydrate, hydroxypropyl cellulose, polyvinyl alcohol (PVA), ethyl cellulose, polyethylene glycol (PEG), talc, triethyl citrate, polyvinyl alcohol-polyethylene glycol copolymers (Kollicoat^{®} IR), ethyl cellulose dispersions (Surelease^{®}), polyvinylprolidone, polyvinylprolidone-vinyl acetate copolymer (PVP-VA), all kinds of Opadry^{®}, pigments, dyes, titanium dioxide, iron oxide or mixtures thereof.

According to one embodiment, the film coating comprises hydroxypropyl methylcellulose, triacetin, glycerol triacetate, talc or mixtures thereof. The amount of the film coating is between 1.0 and 7.0% by weight of the total composition.

According to one embodiment, the enteric coating comprises methacrylic acid / ethyl acrylate copolymer (1:1), talc, titanium dioxide, triethyl citrate, pigments, dyes or mixtures thereof. The amount of the enteric coating is between 3.0 and 10.0% by weight of the total composition.

Analyses conducted regarding this distinctly low ratio range show that the enteric coating maintains its stability on the core tablet and does not dissolve in the gastric environment. Accordingly, the reduced amount of the enteric coating covering the core tablet shall increase patient compliance due to the reduced size of the final pharmaceutical product.

The pharmaceutical bilayer tablet of the present invention may be prepared, using standard techniques and manufacturing processes well known in the art, such as direct compression, wet granulation or dry granulation, hot melt granulation, hot melt extrusion, fluidized bed granulation, extrusion/spheronization, slugging, spray drying and solvent evaporation.

According to one embodiment of the present invention, the first layer is prepared with wet granulation, the second layer is prepared with direct compression. Thanks to the use of wet granulation technique in the first layer, Cyanocobalamin (Vitamin B12), which is in low dose in the tablet, was homogeneously dispersed. Preferring direct compression technique for second layer provides ease of production.

### Example 1: The bilayer tablet formulation

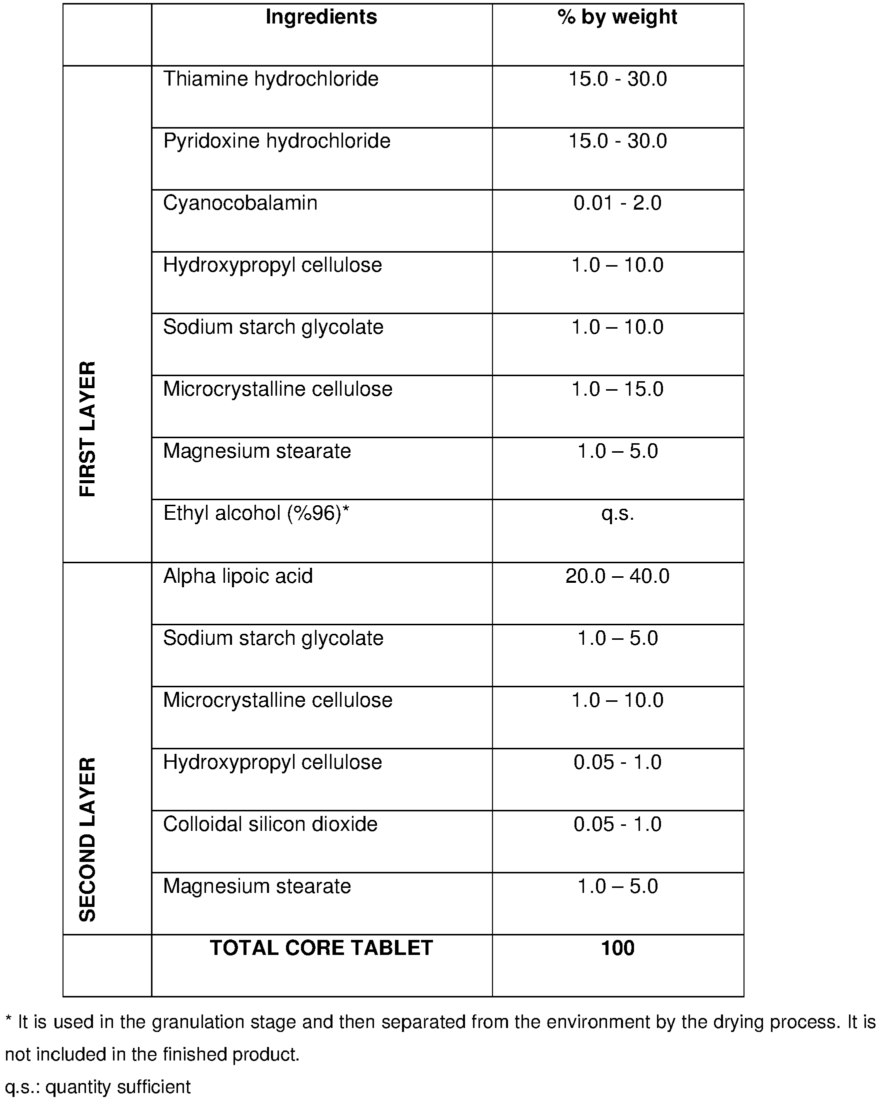

| **Ingredients** | **% by weight** |
|---|---|
| Total core tablet | 85.0 - 96.0 |
| Film coating | 1.0 - 7.0 |
| Enteric coating | 3.0 - 10.0 |
| **TOTAL COMPOSITION** | **100** |

### Example 2: The bilayer tablet formulation

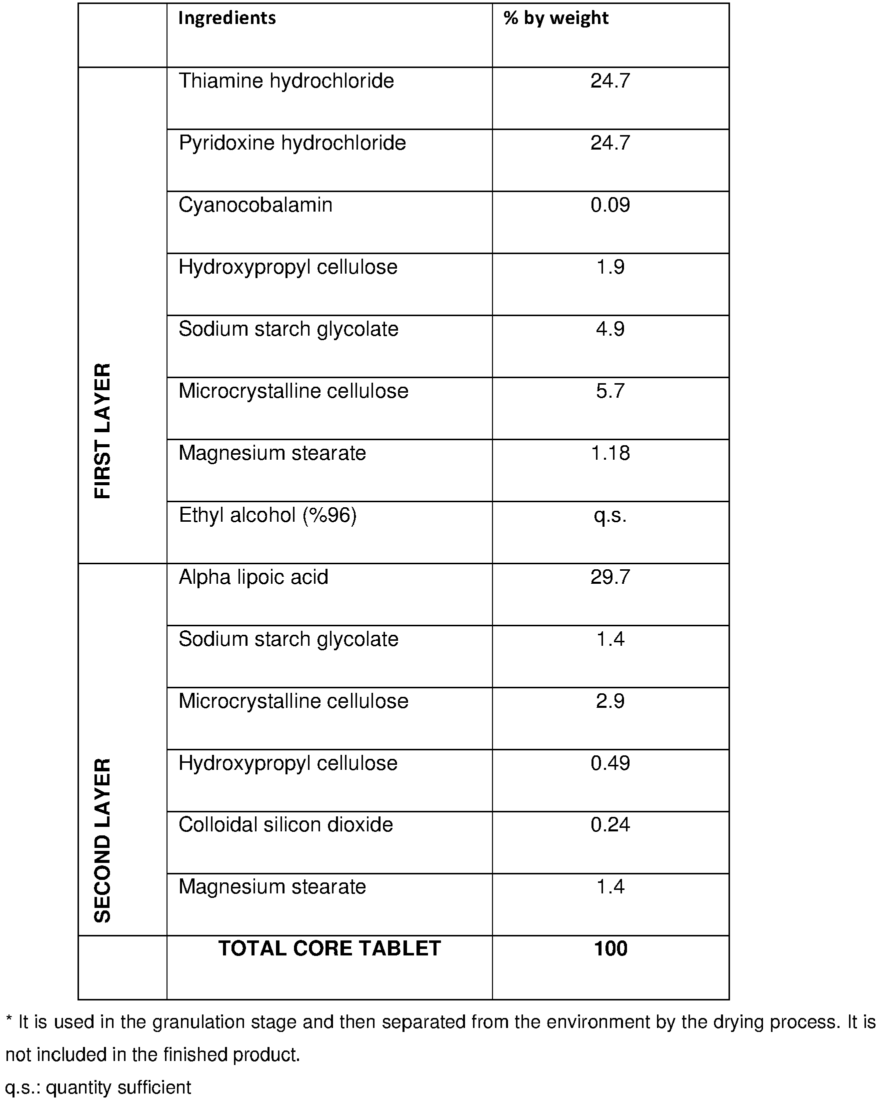

| **Ingredients** | **% by weight** |
|---|---|
| Total core tablet | 92.8 |
| Film coating | 2.0 |
| Enteric coating | 5.20 |
| **TOTAL COMPOSITION** | **100** |

### Example 3: The bilayer tablet formulation

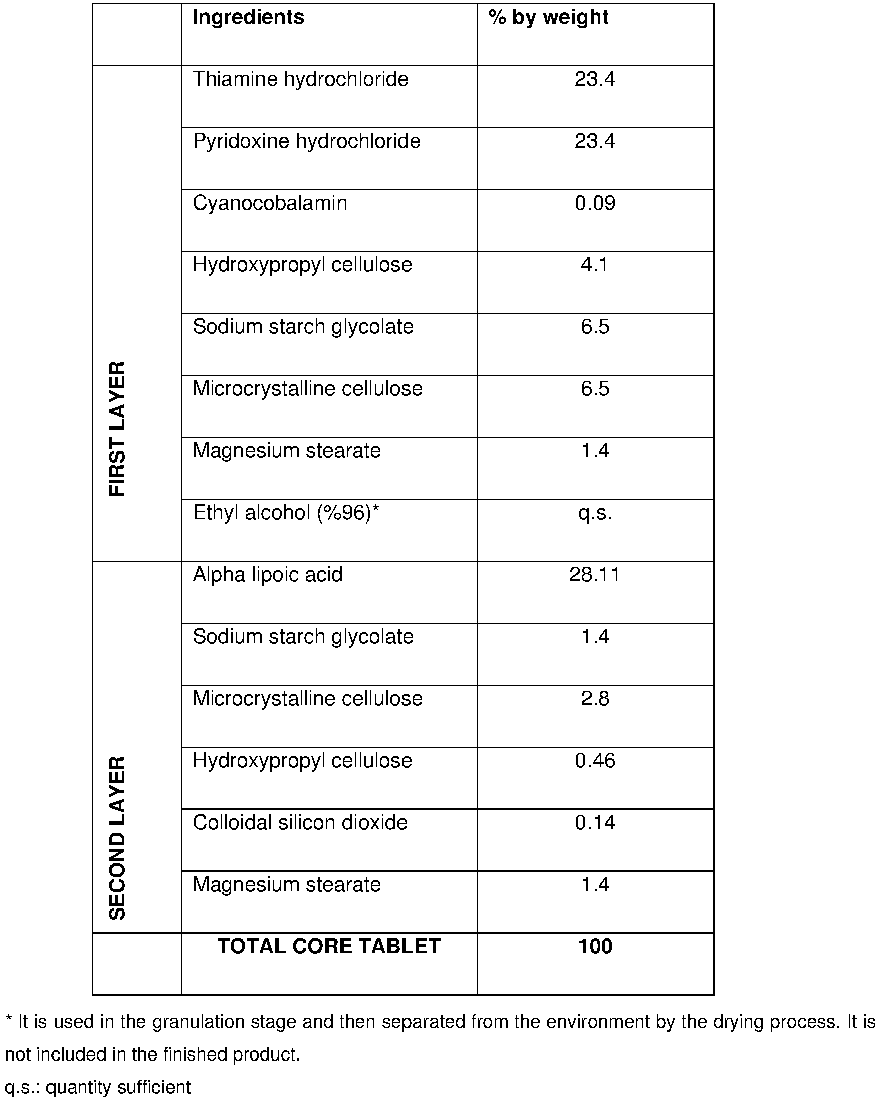

| **Ingredients** | **% by weight** |
|---|---|
| Total core tablet | 92.8 |
| Film coating | 2.0 |
| Enteric coating | 5.20 |
| **TOTAL COMPOSITION** | **100** |

### Process for example 1, example 2 and example 3;

**For the first layer:**
   a) Mixing thiamine hydrochloride, pyridoxine hydrochloride, cyanocobalamin, microcrystalline cellulose, sodium starch glycolate and three-fourths of hydroxypropyl cellulose together to prepare a powder mixture comprising B vitamins,
   b) Mixing one fourth of hydroxypropyl cellulose and ethyl alcohol to prepare a granulation solution,
   c) Granulating the powder mixture comprising B vitamins with the granulation solution (wet granulation),
   d) Sieving the granules comprising B vitamins and drying them in a fluid bed dryer,
   e) Sieving the dried granules again,
   f) Adding magnesium stearate to the sieved granules and mixing this total mixture,
**For the second layer;**
   g) On the other side, sieving alpha lipoic acid, hydroxypropyl cellulose, sodium starch glycolate, colloidal silicon dioxide and microcrystalline cellulose,
   h) Mixing these together to prepare a powder mixture comprising alpha lipoic acid,
   i) Adding magnesium stearate to the powder mixture comprising alpha lipoic acid and mixing the total mixture,

   - Compressing these two total mixtures (step (f) and step (i)) together into bilayer tablets,
   - Preparing a film coating solution and coating the tablets with this solution,
   - Then, preparing an enteric coating solution and coating the tablets with this solution.

### Example 4: The film coating

| **Ingredients** | **% by weight** |
|---|---|
| Hydroxypropyl methylcellulose | 84.1 |
| Triacetin/ glycerol triacetate | 8.4 |
| Talc | 7.5 |
| **TOTAL FILM COATING** | **100** |

### Example 5: The enteric coating

| **Ingredients** | **% by weight** |
|---|---|
| Methacrylic acid / ethyl acrylate copolymer (1:1) | 50.0 |
| Talc | 32.0 |
| Titanium dioxide | 11.75 |
| Triethyl citrate | 6.0 |
| Dyes | 0.25 |
| **TOTAL ENTERIC COATING** | **100** |

## Claims

1. A pharmaceutical bilayer tablet comprising,
a. first layer comprising thiamine hydrochloride, pyridoxine hydrochloride and cyanocobalamin,
b. second layer comprising alpha lipoic acid,
wherein each layer comprising at least one disintegrant and wherein disintegrants are selected from the group of consisting of sodium starch glycolate, low-substitute poloxamer, magnesium aluminum silica, sodium carboxy methyl cellulose, carboxy methyl cellulose calcium, docusate sodium, polyacrylin potassium, sodium glycine carbonate or mixtures thereof
wherein the amount of the disintegrants is between 1.0% and 15.0% by weight of the total core tablet,
wherein the amount of the disintegrants in the first layer is between 1.0% and 10.0% by weight of the total core tablet and the amount of the disintegrants in the second layer is between 1.0% and 5.0% by weight of the total core tablet and,
wherein the disintegrant is sodium starch glycolate.

2. The pharmaceutical bilayer tablet according to claim 1, wherein the weight ratio of first layer to second layer is between 1.0 and 3.0.

3. The pharmaceutical bilayer tablet according to claim 1, wherein a tablet comprising;
- 15.0% - 30.0% by weight of thiamine hydrochloride,
- 15.0% - 30.0% by weight of pyridoxine hydrochloride,
- 0.01% - 2.0% by weight of cyanocobalamin,
- 20.0% - 40.0% by weight of alpha lipoic acid.

4. The pharmaceutical bilayer tablet according to claim 1, wherein the bilayer tablet further comprises at least one pharmaceutically acceptable excipient selected from fillers, binders, lubricants/glidants or mixtures thereof.

5. The pharmaceutical bilayer tablet according to claim 4, wherein at least two same excipients has been used for each layer.

6. The pharmaceutical bilayer tablet according to claim 1, wherein the tablet comprising a core and two coating layers which are a film coating layer and an enteric coating.

7. The pharmaceutical bilayer tablet according to claim 6, wherein the film coating comprising hydroxypropyl methylcellulose, triacetin, glycerol triacetate, talc or mixtures thereof.

8. The pharmaceutical bilayer tablet according to claim 6, wherein the enteric coating comprising methacrylic acid / ethyl acrylate copolymer (1:1), talc, titanium dioxide, triethyl citrate, pigments, dyes or mixtures thereof.

## Patentansprüche

1. Eine pharmazeutische Zweischichttablette, umfassend:
a. eine erste Schicht, umfassend Thiaminhydrochlorid, Pyridoxinhydrochlorid und Cyanocobalamin,
b. eine zweite Schicht, umfassend Alpha-Liponsäure,
wobei jede Schicht mindestens ein Sprengmittel umfasst und wobei Sprengmittel ausgewählt sind aus der Gruppe bestehend aus Natriumstärke-Glykolat, niedrig substituiertem Poloxamer, Magnesium-Aluminium-Silikat, Natriumcarboxymethylcellulose, Carboxymethylcellulose-Calcium, Docusat-Natrium, Polyacrilin-Kalium, Natriumglycincarbonat oder Mischungen davon,
wobei die Menge der Sprengmittel zwischen 1,0 Gew.-% und 15,0 Gew.-% des Gesamttablettenkerns beträgt,
wobei die Menge der Sprengmittel in der ersten Schicht zwischen 1,0 Gew.-% und 10,0 Gew.-% des Gesamttablettenkerns beträgt und die Menge der Sprengmittel in der zweiten Schicht zwischen 1,0 Gew.-% und 5,0 Gew.-% des Gesamttablettenkerns beträgt, und
wobei das Sprengmittel Natriumstärke-Glykolat ist.

2. Die pharmazeutische Zweischichttablette nach Anspruch 1, wobei das Gewichtsverhältnis der ersten Schicht zur zweiten Schicht zwischen 1,0 und 3,0 liegt.

3. Die pharmazeutische Zweischichttablette nach Anspruch 1, wobei eine Tablette umfasst:
- 15,0 Gew.-% - 30,0 Gew.-% Thiaminhydrochlorid,
- 15,0 Gew.-% - 30,0 Gew.-% Pyridoxinhydrochlorid,
- 0,01 Gew.-% - 2,0 Gew.-% Cyanocobalamin,
- 20,0 Gew.-% - 40,0 Gew.-% Alpha-Liponsäure.

4. Die pharmazeutische Zweischichttablette nach Anspruch 1, wobei die Zweischichttablette ferner mindestens einen pharmazeutisch annehmbaren Hilfsstoff umfasst, ausgewählt aus Füllstoffen, Bindemitteln, Schmiermitteln/Fließregulierungsmitteln oder Mischungen davon.

5. Die pharmazeutische Zweischichttablette nach Anspruch 4, wobei für jede Schicht mindestens zwei gleiche Hilfsstoffe verwendet wurden.

6. Die pharmazeutische Zweischichttablette nach Anspruch 1, wobei die Tablette einen Kern und zwei Überzugsschichten umfasst, welche eine Filmüberzugsschicht und ein magensaftresistenter Überzug sind.

7. Die pharmazeutische Zweischichttablette nach Anspruch 6, wobei der Filmüberzug Hydroxypropylmethylcellulose, Triacetin, Glycerintriacetat, Talkum oder Mischungen davon umfasst.

8. Die pharmazeutische Zweischichttablette nach Anspruch 6, wobei der magensaftresistente Überzug Methacrylsaure-Ethylacrylat-Copolymer (1:1), Talkum, Titandioxid, Triethylcitrat, Pigmente, Farbstoffe oder Mischungen davon umfasst.

## Revendications

1. Comprimé bicouche pharmaceutique comprenant :
a. première couche comprenant du chlorhydrate de thiamine, du chlorhydrate de pyridoxine et de la cyanocobalamine,
b. seconde couche comprenant de l'acide alpha-lipoïque,
dans lequel chaque couche comprend au moins un désintégrant et dans lequel les désintégrants sont choisis dans le groupe constitué par le glycolate d'amidon sodique, un poloxamère faiblement substitué, le silicate de magnésium et d'aluminium, la carboxyméthylcellulose sodique, la carboxyméthylcellulose calcique, le docusate de sodium, le polyacrilide potassique, le carbonate de glycine sodique ou leurs mélanges,
dans lequel la quantité des désintégrants est comprise entre 1,0 % et 15,0 % en poids du noyau de comprimé total,
dans lequel la quantité des désintégrants dans la première couche est comprise entre 1,0 % et 10,0 % en poids du noyau de comprimé total et la quantité des désintégrants dans la seconde couche est comprise entre 1,0 % et 5,0 % en poids du noyau de comprimé total et,
dans lequel le désintégrant est le glycolate d'amidon sodique.

2. Comprimé bicouche pharmaceutique selon la revendication 1, dans lequel le rapport pondéral de la première couche à la seconde couche est compris entre 1,0 et 3,0.

3. Comprimé bicouche pharmaceutique selon la revendication 1, dans lequel un comprimé comprend :
- 15,0 % - 30,0 % en poids de chlorhydrate de thiamine,
- 15,0 % - 30,0 % en poids de chlorhydrate de pyridoxine,
- 0,01 % - 2,0 % en poids de cyanocobalamine,
- 20,0 % - 40,0 % en poids d'acide alpha-lipoïque.

4. Comprimé bicouche pharmaceutique selon la revendication 1, dans lequel le comprimé bicouche comprend en outre au moins un excipient pharmaceutiquement acceptable choisi parmi les charges, les liants, les lubrifiants/agents de glissement ou leurs mélanges.

5. Comprimé bicouche pharmaceutique selon la revendication 4, dans lequel au moins deux mêmes excipients ont été utilisés pour chaque couche.

6. Comprimé bicouche pharmaceutique selon la revendication 1, dans lequel le comprimé comprend un noyau et deux couches d'enrobage qui sont une couche de pelliculage et un enrobage entérique.

7. Comprimé bicouche pharmaceutique selon la revendication 6, dans lequel le pelliculage comprend de l'hydroxypropylméthylcellulose, de la triacétine, du triacétate de glycérol, du talc ou leurs mélanges.

8. Comprimé bicouche pharmaceutique selon la revendication 6, dans lequel l'enrobage entérique comprend un copolymère d'acide méthacrylique / acrylate d'éthyle (1:1), du talc, du dioxyde de titane, du citrate de triéthyle, des pigments, des colorants ou leurs mélanges.
